(19) **Europäisches Patentamt** / **European Patent Office** / **Office européen des brevets**

(11) **EP 3 880 883 B1**

(12) **EUROPEAN PATENT SPECIFICATION**

(45) Date of publication and mention
of the grant of the patent:
**25.10.2023 Bulletin 2023/43**

(51) International Patent Classification (IPC):
**D21H 11/18** (2006.01)     **A23L 29/262** (2016.01)
**C08B 15/08** (2006.01)     **C08L 1/02** (2006.01)

(21) Application number: **19809556.4**

(52) Cooperative Patent Classification (CPC):
**D21H 11/18; A23L 29/262; C08B 15/02;
C08L 1/02; C08L 97/02**

(22) Date of filing: **13.11.2019**

(86) International application number:
**PCT/GB2019/053222**

(87) International publication number:
**WO 2020/099872 (22.05.2020 Gazette 2020/21)**

(54) **METHOD FOR PROCESSING FIBROUS CELLULOSIC MATERIAL, PRODUCTS AND USES THEREOF**

VERFAHREN ZUR VERARBEITUNG VON FASERIGEM ZELLULOSEMATERIAL, PRODUKTE UND VERWENDUNGEN DAVON

PROCÉDÉ DE TRAITEMENT DE MATIÈRE CELLULOSIQUE FIBREUSE, PRODUITS ET UTILISATIONS S'Y RAPPORTANT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **13.11.2018 GB 201818498**

(43) Date of publication of application:
**22.09.2021 Bulletin 2021/38**

(73) Proprietor: **The Court Of Edinburgh Napier
University
Edinburgh
Lothian EH4 1DJ (GB)**

(72) Inventors:
• **O'ROURKE, Dominic
Dundee DD2 1DA (GB)**
• **DORRIS, Mark
Strathaven ML10 6AN (GB)**

(74) Representative: **Murgitroyd & Company
Murgitroyd House
165-169 Scotland Street
Glasgow G5 8PL (GB)**

(56) References cited:
**CN-A- 103 255 662     CN-A- 106 267 370**

**US-A1- 2009 317 437     US-A1- 2014 154 756
US-A1- 2017 150 749**

• **XIANG ZHOUYANG ET AL: "A comparison of cellulose nanofibrils produced fromCladophora glomerataalgae and bleached eucalyptus pulp", CELLULOSE, SPRINGER NETHERLANDS, NETHERLANDS, vol. 23, no. 1, 22 December 2015 (2015-12-22), pages 493-503, XP035904015, ISSN: 0969-0239, DOI: 10.1007/S10570-015-0840-7 [retrieved on 2015-12-22]**
• **KAI HUA ET AL: "Translational study between structure and biological response of nanocellulose from wood and green algae", RSC ADVANCES, vol. 4, no. 6, 1 January 2014 (2014-01-01), pages 2892-2903, XP055664556, UK ISSN: 2046-2069, DOI: 10.1039/C3RA45553J**
• **JIAQI GUO ET AL: "Contribution of Residual Proteins to the Thermomechanical Performance of Cellulosic Nanofibrils Isolated from Green Macroalgae", ACS SUSTAINABLE CHEMISTRY & ENGINEERING, vol. 5, no. 8, 7 August 2017 (2017-08-07), pages 6978-6985, XP055664617, US ISSN: 2168-0485, DOI: 10.1021/acssuschemeng.7b01169**

EP 3 880 883 B1

- Moon Robert J. ET AL: "Cellulose nanomaterials review: structure, properties and nanocomposites", Chemical Society Reviews, vol. 40, no. 7, 1 January 2011 (2011-01-01), page 3941, XP055945221, UK ISSN: 0306-0012, DOI: 10.1039/c0cs00108b

## Description

## Technical Field

The present invention relates to a method for processing fibrous cellulosic material, cellulose nanofibrils resulting from such method and use of the resulting cellulose nanofibrils.

## Background of the Invention

Cellulosic material which consists of at least one dimension in the range of less than 1 micron, typically less than 500nm, is known as nanocellulose.

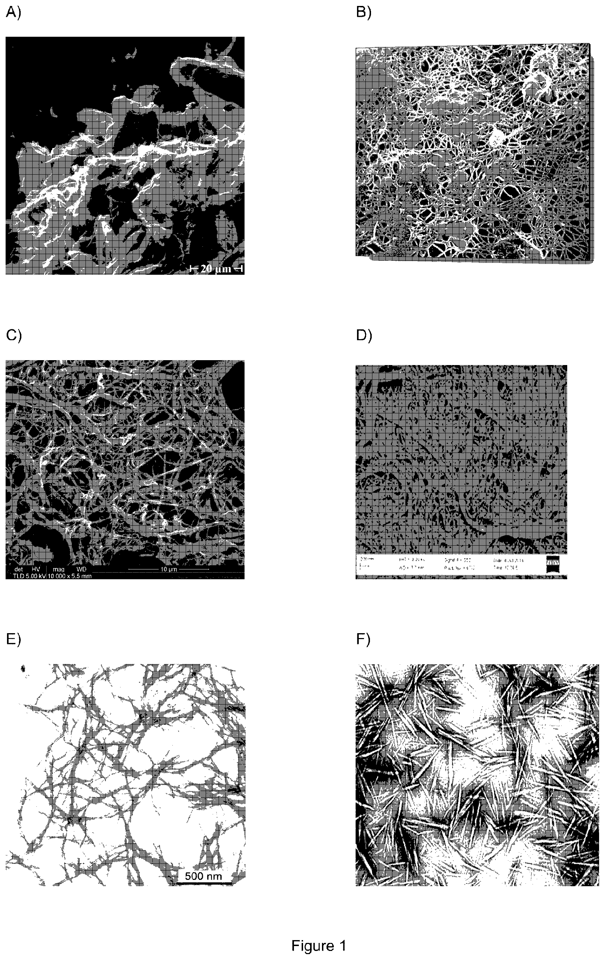

Examples of nanocellulose, as illustrated in Figure 1, include microcrystalline cellulose (MMC; Fig. 1A), cellulose microfibrils (CMF; Fig. 1B), microfibrillated cellulose (MFC; Fig. 1C and NFC; Fig. 1D), cellulose nanofibrils (CNF; Fig. 1E) and cellulose nanocrystals (CNC; Fig. 1F). The present invention relates to a method of processing fibrous cellulosic material into cellulose nanofibrils, which are the smallest type of cellulose still in fibril state, and cellulose nanocrystals, which is the smallest type of cellulose available (typically 100s to 1000 nanometers) and is highly crystalline and rigid nanoparticles. CNF has a high aspect ratio (length to width ratio) with typical widths of 5 to 20 nanometers and a wide range of lengths, typically of several micrometers.

Sources of cellulose feedstock include bacteria, algae and higher plants, i.e. land plants with lignified tissues for transporting water and minerals through the plant.

Commercial production of nanocellulose is derived from wood pulp, often as a by-product of the paper industry. In particular, mechanical methods are used to isolate the nanocellulose fibres from the wood-based fibres by exposing the pulp to high shear force. Typically, high-pressure homogenisers, ultrasonic homogenisers, grinders or microfluidizers are employed which delaminate the cell walls in the wood pulp in order to extract the nanocellulose. Such a process has high energy consumption for low product yields as the starting cellulose concentrations are required to be less than 0.5% by weight, typically about 0.2% by weight, and so are relatively expensive with respect to the end product.

Pre-treatments, such as enzymatic or mechanical, or introduction of charged species via carboxymethylation or TEMPO-mediated oxidation, have been proposed to reduce energy consumption and therefore cost. However, such additional steps will add further costs to the procedure which may reduce or eliminate any cost savings resulting from the reduced energy consumption.

Alternatively, nanocellulose may be derived from bacterial cellulose, which produces a high value end product but involves high cost processing and so is primarily intended for high value end use such as biomedical applications.

Nanocellulose crystals are typically formed by further processing of the native fibrils by acid hydrolysis with sulphuric or hydrochloric acid. In particular, the nanofibrils are hydrolysed and subsequently CNC is extracted from the acid solution by centrifugation and washing. However, CNF derived from wood is not readily amenable to such acid hydrolysis techniques, leading to typical yields of less than 30%. Therefore, such methods are not considered to be economically viable where a low value end product is desired.

Nanocellulose has wide ranging applications including composites, oil and gas, paints, cosmetics, foods, coatings and films. Depending upon the nature of the nanocellulose, its use ranges from lower value rheology modifiers to high value medical composites. CMF, CNF and CNC are typically the desired end product in cellulose processing for most applications.

JIAQI GUO ET Al, Contribution of Residual Proteins to the Thermomechanical Performance of Cellulosic Nanofibrils Isolated from Green Macroalgae, ACS Sustainable Chemistry & Engineering, vol. 5, no. 8, 7 August 2021, pages 6978-6985 and MOON ROBERT J. ET AL, Cellulose Nanomaterials Review: Structure, Properties and Nanocomposites, Chemical Society Reviews, vol. 40, no. 7, 1 January 2011, page 3941 disclose processing of nanocellulose materials from green algae.

There exists a need to provide a more cost efficient method for producing nanocellulose which negates the need to use high energy consumption processes or additional processing steps.

## Summary of the Invention

In a first aspect of the invention there is provided a method for processing fibrous cellulosic material from brown seaweed as defined in claim 1.

It has not previously been possible to process fibrous cellulosic material in water alone via high shear homogenisation due to the potential for the fibrous material to block chambers and cause excessive wear to steel components. However, the inventors have surprisingly found that the combination of less aggressive handling and processing conditions, namely processing in water in less intense conditions, through at least one chamber with a larger gap for one or more passes, enables a simplified procedure for processing fibrous cellulosic material into nanocellulose. For example, a single pass through a chamber with a 200 micron gap at relatively low pressure has been found to produce discrete cellulose nanofibrils of 40 to 100nm in width and with an aspect ratio of greater than 50. The resulting cellulose nanofibrils are a pure product (typically 99.9% yield) of high quality.

This single high shear processing step reduces energy costs and $CO_2$ emissions dramatically compared to other high shear nanocellulose processing methods. Further, the single processing step allows for modular bolt-on technology to existing seaweed extraction processes.

In addition, the method of the present invention produces no significant waste steam as typically 99.9% nanocellulose yields are achieved. The process uses only water and not any hazardous substances, thus requiring no special training or personal protective equipment.

Further, the use of a low pressure pass for high shear processing considerably reduces down time in processing due to a reduction in cavitation effects and mechanical wear typical for higher plant derived high shear nanocellulose processing, which may require many high pressure passes.

The resulting cellulose nanofibrils are very durable and do no undergo any degradation regardless of the number of passes through the at least one chamber having a large gap at high shear. Further, the resulting cellulose nanofibrils are more readily amenable to acid hydrolysis to form cellulose nanocrystals (as discussed in further detail below) and to surface modification than cellulose nanofibrils derived from other sources, such as wood pulp.

In particular, the present invention makes use of fibrous cellulosic material derived from algae. Algae is not a common feedstock for nanocellulose production as it is not considered to be commercially viable due to the low cellulose content (typically around 10wt%) and high cost of the methods known in the art. Therefore, the present invention enables fibrous cellulosic material derived from algae to be processed at lower cost, thus making algae-derived nanocellulose production a commercially viable option.

The term "algae" is intended to include all fibrous cellulose containing algae, including but not limited to microalgae and seaweed. The algae is brown seaweed in the present invention.

Fibrous cellulosic material is a by-product in the processing of seaweed, for example, processing of brown seaweed to form alginate. Such a material is present in varying quantities and is not bound to any other fragments which may render it difficult to break down, thus allowing extraction by the method of the present invention.

Brown seaweed typically has a comparatively high cellulose content, for example *Laminaria hyperborea* comprises 10 to 12 % cellulose which is present in the by-product of the extraction of alginate. The cellulose content of brown seaweed is higher than in other algae and so provides the starting material for the present invention.

The cellulosic fibrous material are extracted from algae together with alginate and subsequently separated from alginate to provide an isolated cellulosic fibrous starting material. Separation may be achieved by filtration of the insoluble cellulosic fibrous material from the soluble alginate in water.

The cellulosic fibrous material is not subjected to any pre-treatments to aid extraction of the cellulose nanofibrils such as treatments with swelling, hydrolytic or oxidation agents prior to suspending in water, as are required in known cellulose nanofibril production from wood pulp or the like.

The method of the present invention enables higher concentrations of fibrous cellulose material to be processed. For example, the fibrous cellulosic material may be suspended in water in a concentration up to 5% by weight. As will be appreciated, the inherent rheological properties of the material are a limiting factor to the concentration in water and so the concentration may be selected to ensure that the resulting suspension is not too viscous. For example, a concentration in the range of 0.5 to 5 % by weight may be used. Alternatively, concentrations of 0.5 to 4 % by weight or 1 to 3 % by weight may be used. Concentrations less than 0.5 % by weight are of course possible, for example, very low concentrations of 0.1 wt% and 0.2 wt% may be processed this way including with some wood derived cellulose materials. However, the process may not be commercially viable at such low concentrations.

The at least one chamber may be any suitable configuration, for example, Z or Y shaped or straight as per auxiliary chambers. For example, the at least one chamber may be an interaction chamber and/or an auxiliary chamber.

The suspension passes through the at least one gap into the at least one chamber for processing therein. The at least one gap is from 70 micron to 250 microns. If at least one chamber having a smaller sized gap (i.e. up to 100 microns) is employed, the suspension may be required to undergo multiple passes through the chamber (for example, up to 7) in order to achieve a highly homogeneous product. However, where at least one chamber having a gap of 200 microns is employed, a similarly pure product may be achieved with one pass through the chamber. Therefore, the gap size may be selected accordingly, although it is appreciated that processing down time may be increased if a smaller size gap is used due to blockages. In one or more embodiments, the at least one chamber incorporating a single gap may be used.

The suspension undergoes multiple passes through the at least one chamber having a large gap. The number of passes is up to 7 passes. In some embodiments, the number of passes may be up to 5 passes or up to 3 passes.

The suspension may be passed through the at least one chamber at a comparatively low pressure compared to known high shear nanocellulose processing. Typical pressure employed in the second step of the method of the present invention may be up to 200 MPa. For example, pressures of up to 140 MPa may be used for a chamber having a 200 micron gap, whereas pressures of up to 200 MPa may be used for a chamber having a gap of 70 to 100 microns.

Processing of the cellulosic fibrous material to nanofibrils may be achieved via the method of the present invention consisting only of the steps described above. Indeed, high quality cellulose nanofibrils in high yields may readily be achieved with only a single chamber as described above. However, in some situations, multiple chambers may be employed.

The water may subsequently be removed from the suspension comprising the cellulose nanofibrils to produce cellulose nanofibrils in a dry form. This may be achieved by any suitable means in the art, for example, freeze drying, spray drying, supercritical drying or by evaporation through solvent exchange into acetone or tert-butanol in a first step. However, any methods which leads to agglomeration, such as air drying in a hot oven, should be avoided.

The method of the present invention is carried out in a high shear homogeniser. Any commercially available high shear homogeniser may be adapted to carry out the method of the present invention.

The method of the present invention may further comprise subjecting the cellulose nanofibrils to acid hydrolysis to form cellulose nanocrystals.

Acid hydrolysis is a standard technique in the art for forming cellulose nanocrystals. However, it has been found that the cellulose nanofibrils formed by the present invention are more amenable to acid hydrolysis than CNF from other sources. Consequently, the resulting yield of cellulose nanocrystals is much higher than for wood-derived cellulose, for example, at least a two-fold increase in yield. Further, the resulting nanocrystals are a high quality end product comparable in size and morphology to bacterial synthesised cellulose nanocrystals but at much lower cost. In comparison, cellulose nanocrystals derived from wood pulp are much smaller and of inferior quality.

As with the cellulose nanofibrils, the resulting nanocrystals are in an easily separable form, ready for use in a wide range of applications.

Acid hydrolysis may use hydrochloric acid, sulphuric acid, nitric acid, phosphoric acid or the like. In one or more embodiments, the acid hydrolysis uses hydrochloric acid. It has been found that the use of hydrochloric acid results in at least a 2.3-fold increase in the yield of cellulose nanocrystals derived from cellulose nanofibrils formed from the method of the present invention.

The method may further comprise one or more steps in which the cellulose nanofibrils or cellulose nanocrystals are adapted for specific applications. For example, the cellulose nanofibrils or cellulose nanocrystals may be subjected to conditions in which a change in surface chemistry is effected to impart desired functionality, for instance, introducing a surface charge by reaction with an oxidising agent and/or an acid. In one such step the cellulose nanofibrils or cellulose nanocrystals may undergo surface modification either subsequent or prior to their production. Such steps are known for CNF and CNC derived from other sources and can be readily applied to the products of the present invention before or after processing by the method of the present invention.

As is known in the art, oxidising agents or acids (which may also be oxidising agents) break down amorphous regions of the cellulose nanofibrils leaving the cellulose nanocrystals. The reaction time determines the end product: if short reaction times are used, surface modified CNF results; if long reaction times are used, CNC or modified CNC results, depending upon the acid/oxidising agent used; if the oxidisation is allowed to reach completion, whether the CNC is broken down into sugar crystals. In particular, where strong acids such as hydrochloric acid or sulphuric acid are used, the reaction is fast, reaching complete hydrolysis in, for example 1 to 4 hours. Therefore, in order to achieve surface modified CNF, reactions times in the order of minutes are required. Further, ammonium persulphate may provide the fastest and cheapest way of oxidised CNF, typically with a one-hour reaction time. However, it may be preferable to use weaker acids such as citric acid, acetic acid, nitric acid or phosphorus acid for more controlled surface modification of CNF, without significant amounts of CNC, which may typically have reaction times of 12 hours. Further control may be achieved by conducting the surface modification either before or after processing of the fibrous cellulose material to CNF. For example, oxidation on coarse fibrous cellulose material may be less effective than oxidation on the resulting CNF but may limit unwanted hydrolysis to CNC. Therefore, the skilled person can readily select a reaction time depending upon the desired product and the acid/oxidising agent selected and further whether the reaction is completed before or after processing of the fibrous cellulose material to cellulose nanofibrils.

In a second aspect of the present invention, there is provided cellulose nanofibrils as defined in claim 10.

Commercially available material described as cellulose nanofibrils typically comprise a mixture of different sized fibrils and particles with an average size in the nanofibrils range. The cellulose nanofibrils formed by the method of the first aspect of the present invention are a higher quality homogeneous product with a much narrower size distribution within the nano range.

The cellulose nanofibrils is a substantially homogeneous product. As a substantially homogeneous product, at least 95%, for example, at least 97% and in some cases, at least 99% of the cellulose nanofibrils exist as discrete entities each with two dimensions in the nano range.

The cellulose nanofibrils have an aspect ratio of greater than 100.

The unmodified cellulose nanofibrils resulting from the method of the first aspect of the invention may have a water retention of greater than 5000%, for example greater than 6000% or greater than 7000%. A water retention of 5000% means that the material will absorb 50 times its own weight in water, 6000% means 60 times its own weight and 7000% means 70 times its own weight in water. CNF optionally oxidised by citric acid, acetic acid, nitric acid or phosphoric acid may have a water retention of greater than 25,000%, which means the material will absorb 250 times its own weight in water. Water retention is particularly desirable where the cellulose nanofibrils are used as a rheological modifier in applications in which remaining wetter for longer is desirable, such as moisturisers, barrier creams, plasters, fillers and wound care products. In particular, oxidised CNF with very high water retention values, for example greater than 25000%, compares directly with fossil fuel derived super absorbent polymers used in industry as thickeners, stabilisers and emulsifiers for personal care and pharmaceutical applications.

Also described are cellulose nanocrystals formed by the method of the present invention.

In a third aspect of the present invention, there is provided a rheological modifier comprising at least one of the cellulose nanofibrils of the second aspect of the present invention.

In a fourth aspect of the present invention, there is provided a medical composite comprising at least one of the cellulose nanofibrils of the second aspect of the present invention.

In a fifth aspect of the present invention, there is provided a single use plastic replacement product comprising at least one of the cellulose nanofibrils of the second aspect of the present invention.

The single use plastic may be films, microbeads, straws or Q tips etc.

In a sixth aspect of the present invention, there is provided a medical implant or a medical mesh comprising at least one of the cellulose nanofibrils of the second aspect of the present invention.

In a seventh aspect of the present invention, there is provided a method of producing a medical implant or a medical mesh comprising 3-D printing of at least one of the cellulose nanofibrils of the second aspect of the present invention.

The 3-D printing may be achieved by any suitable method known in the art.

In an eighth aspect of the present invention, there is provided use of the cellulose nanofibrils of the second aspect of the present invention in composites, oil and gas, paints, cosmetics, foods, coatings or films.

The features of one of the aspects of the present invention apply mutatis mutandis to the other aspects of the present invention.

## Detailed Description

Embodiments of the present invention will now be described with reference to the following, non-limiting examples and figures.

Figure 1 illustrates SEM images of the different types of nanocellulose;
Figure 2 graphically depicts the comparison of storage modulus of fibrous cellulosic material harvested from different origins, processed in accordance with an embodiment of the present invention;
Figure 3 illustrates SEM images for the processed fibrous cellulosic material harvested from the different origins;
Figure 4 illustrates FTIR analysis of the processed fibrous cellulosic material harvested from the different origins;
Figure 5 illustrates SEM images of nanocellulose at the start and end of processing via a method in accordance with a first embodiment of the present invention;
Figure 6 illustrates comparative SEM images of cellulose nanofibrils results from the method of the first embodiment and cellulose nanofibrils derived from wood pulp;
Figure 7 illustrates an SEM image of cellulose nanocrystals formed by the method in accordance with a second embodiment of the present invention;
Figure 8 illustrates SEM images of cellulose nanocrystals derived from sources of nanocellulose other than algae; and
Figure 9 illustrates redispersible stable suspensions of cellulose nanocrystals.

In accordance with one embodiment of the first aspect of the present invention, the steps for the production of nanofibrillated cellulose (CNF) from coarse cellulose extracted from brown algae are described below. In particular, the fibrous cellulosic material processed in the method of this embodiment was derived from brown seaweed species *Laminaria hyperborea.*

Source of fibrous cellulosic material

Fibrous cellulosic material was obtained from three sources of *Laminaria hyperborea*: the first two sources were by-products of alginate production obtained from Marine Biopolymers Ltd who extracted the fibrous cellulosic material with alginate from seaweed harvested from the Scotland (Ayr) and from Iceland and then separated the two products; and the third source was obtained by harvesting L. *hyperborea* directly from Ayr beach in Scotland by Edinburgh Napier University (ENU) and extracting the coarse cellulose in the laboratory as per the procedure below.

*Seaweed extraction procedure*

1. *Laminaria hyperborea* stems cut into small pieces;
2. Soak overnight in 0.2M HCl;
3. Neutralise to pH 7 with NaOH and then water wash in centrifuge;
4. Soak the resultant material for 4 hours and 30 minutes in 2% sodium bicarbonate; and
5. Centrifuge to remove alginate.

The extracted fibrous cellulosic material from each source was subjected to the method of the present invention in accordance with the embodiment described in further detail below to produce nanofibrillated cellulose (CNF).

A comparison of rheology (storage modulus) and scanning electron microscopy (SEM) analyses of the resulting CNF and Fourier Transform Infra-Red (FTIR) analysis of the extracted fibrous cellulosic material harvested from the above sources was undertaken. The various methods of analysis were intended to establish whether the source of the fibrous cellulosic material and method of extraction affect the nature of the starting material and the subsequent processing into CNF in accordance with the method of the present invention.

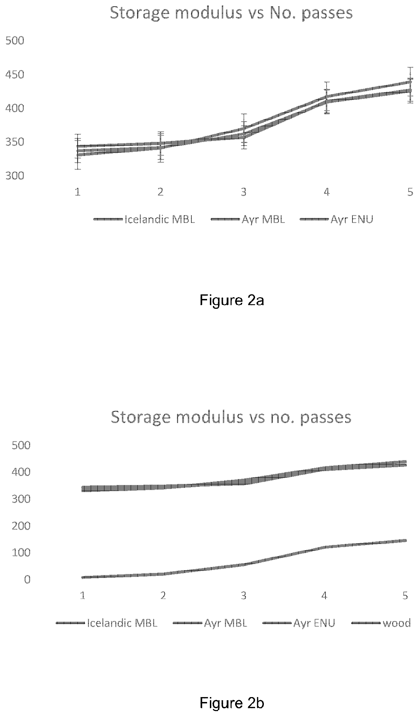

Storage modulus is a measure of the elastic response of a material and refers to the stored energy within it. The results for the resulting CNF from the different sources of fibrous cellulosic material are provided in Table 1 below and in Figure 2a.

Table 1:

| Number of Passes in high shear homogeniser | Storage modulus @50 radls (Pa) Icelandic LHCNF Harvested by MBL | SD | Storage modulus @50 radls (Pa) Scottish LHCNF Harvested by MBL | SD | Storage modulus @50 radls (Pa) Scottish LHCNF Harvested by ENU | SD |
|---|---|---|---|---|---|---|
| 1 | 330.7 | 4.9 | 343.7 | 25.6 | 337.3 | 12.4 |
| 2 | 341 | 10.4 | 347.7 | 2.1 | 341.7 | 6.2 |
| 3 | 370 | 8 | 356.7 | 4.2 | 362.2 | 9.6 |
| 4 | 417.5 | 0.7 | 410.3 | 10.1 | 409 | 1.7 |
| 5 | 439 | 7.1 | 427.3 | 9.5 | 425.3 | 16.3 |

From the results in Table 1 and Figure 2a, it is evident that all three samples of seaweed derived CNF show similar profiles with no significant differences at each pass through the chamber having a large gap.

When compared to wood derived cellulose, processed after swelling in morpholine, the difference between seaweed derived CNF and wood derived CNF is evident, with a single pass of seaweed material two to three times the storage modulus of five passes with wood derived material, as illustrated in Figure 2b.

The shape of the graphs is typical of processing cellulose in this embodiment of the present invention, with increase in storage modulus most evident after three passes and levelling off after five passes. Storage modulus increases with entanglement of fibres supported through hydrogen bonding between and within the fibres. The values are much higher with seaweed derived material since the fibres are much longer and therefore entanglement is greater. After five passes the hydrogen bonding between the fibres is more easily broken and so storage modulus does not increase further to any great extent. While the storage modulus increases with number of passes for seaweed CNF, the benefits of slightly higher storage modulus may not outweigh the energy costs in increasing number of passes.

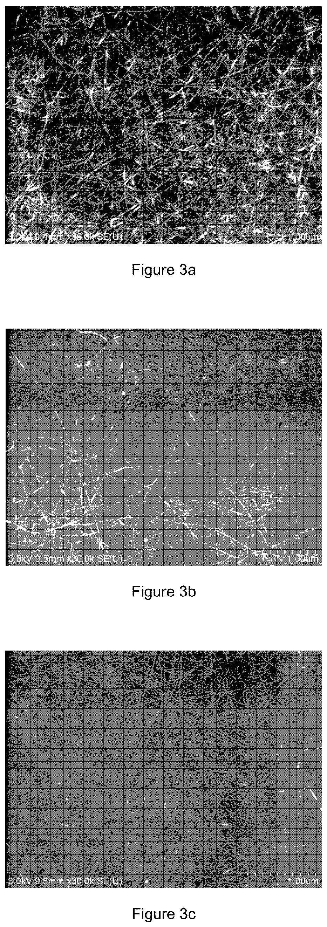

Scanning electron images (SEMs) of CNF material after 1 pass are shown in Figure 3, in which Figure 3a shows Icelandic derived LHCNF from MBL extraction, Figure 3b shows Scottish derived CNF from MBL extraction, and Figure 3c shows Scottish derived CNF from ENU extraction described above. From these images it is clear that there is no discernible differences between the samples.

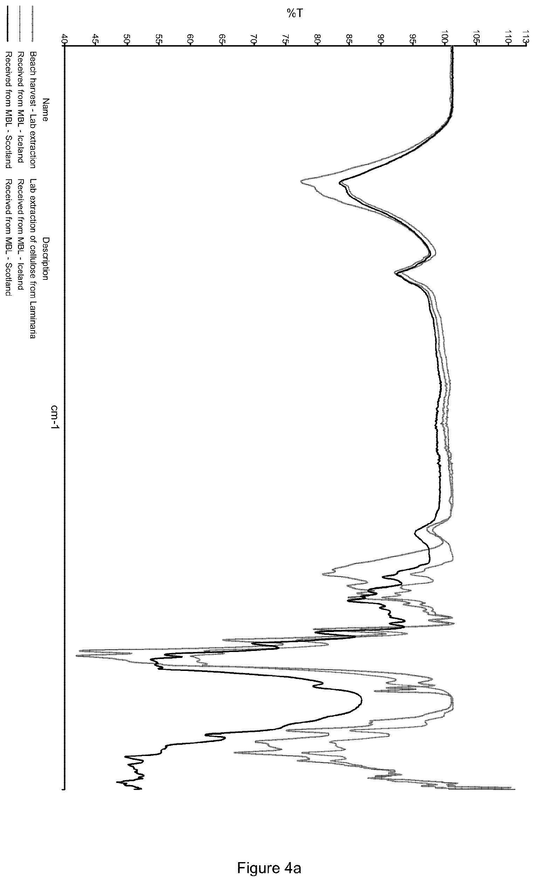

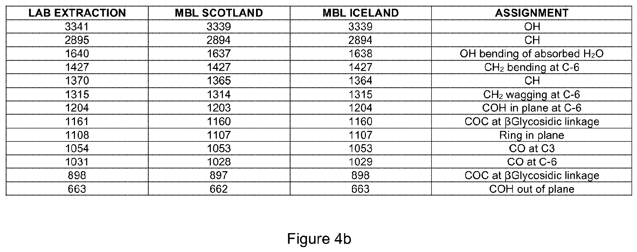

The FTIR (Fourier Transform Infra-Red) traces illustrated in Figure 4a, and quantified in Figure 4b, for the fibrous cellulosic material harvested from all three samples are very similar, showing a pattern expected from seaweed derived cellulose. The ENU and Icelandic samples appear to be the most similar, although no differences are significant between any of the samples i.e. known peaks are where they are expected, with no unexpected peaks. Accordingly, the fibrous cellulosic harvested from the different sources and extracted by different methods (as a by-product of alginate production or via the ENU method) is essentially identical.

Taken together, the results from rheological, SEM imaging, and FTIR analyses show no significant differences between any of the three CNF samples at each pass or three fibrous cellulosic material samples, i.e. in coarse pre-processed form. Therefore, it is apparent that the source of fibrous cellulosic material from algae and method of extraction are not significant to the subsequent CNF processing in accordance with the method of the present invention.

The fibrous cellulosic material starting material used in the subsequent examples is that obtained from MBL, harvested from Scotland and extracted as a by-product of alginate production.

Method of production of cellulose nanofibers (CNF) and cellulose nanocrystals (CNC)

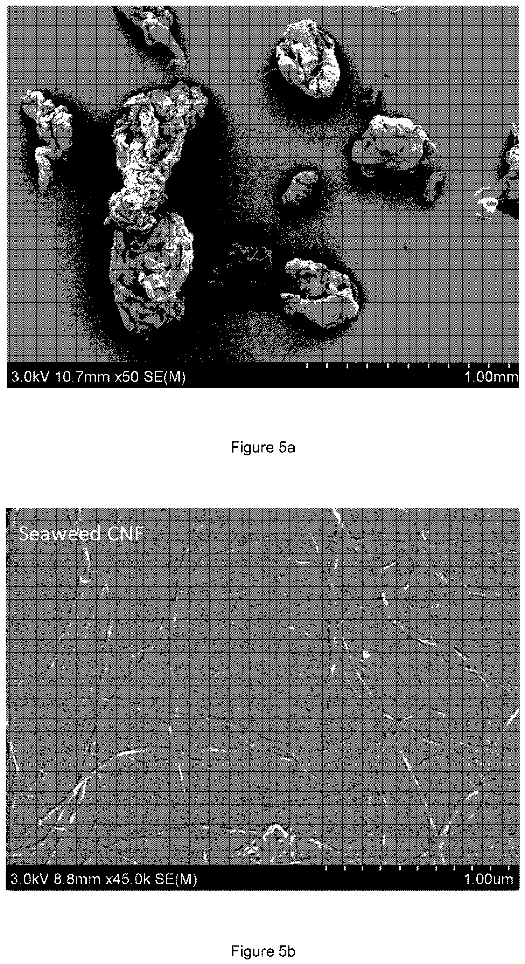

An SEM image of the fibrous cellulosic starting material is provided in Figure 5a which shows cellulose particles in the millimetre size range, i.e. $\times 10^6$ order of magnitude greater than the target fibrils. A 500g suspension of 1% w/w fibrous cellulosic material in deionised water was made up.

A high shear mixer with a 200 micron auxiliary chamber was used in this example. In particular, a M110EH-30 (Microfluidics) high shear mixer was employed in which the 100 micron interaction chamber had been removed.

The fibrous cellulosic material suspension was added to the reservoir of the high shear mixer and mixed to maintain a stable suspension. Mixing may be performed manually with a stirring rod or with a rotor stator mixer, for example an IKA T25 ultra-turrax high speed homogeniser.

The higher shear mixer was then turned on and the fibrous cellulosic material suspension was passed through the auxiliary chamber once and the product was collected. In this example, the flow rate was around 8 to 10L/hr and so the product was collected in around 3 to 5 minutes. The default pressure in the chamber was 9Kpsi (approx. 62 MPa).

The resulting product for a stable suspension of fibrous cellulosic material is high quality cellulosic nanofibrils, as detailed below. A SEM image of the resulting cellulose nanofibrils is provided in Figure 5b which clearly show elongated fibrils having widths within the nano range.

The water retention value (%) of the resulting cellulose nanofibrils was investigated. The water retention value provides an indication of the ability of the fibrils to take up water and swell and therefore the ability to act as a rheology modifier. However, it is also reported to be an indication of the degree of fibrillation of a material ("Microemulsion Systems for Fiber Deconstruction into Cellulose Nanofibrils", Carrillo et al., ACS Applied Materials and Interfaces 6, 22622-22627, 2014).

The water retention value was determined by the following procedure:

    1. For dried CNF powders:

        1.1. Weight 0.5g of dried powder in disposable plastic bottle
        1.2. Add 49.5g of pure water in the bottle
        1.3. Re-disperse the sample by any known methods, for example using a rotor stator mixer, sonication or a further pass through the high shear homogeniser.
        1.4. Place the suspension in a 50ml falcon tube

    2. For never dried cellulosic material in solvent other than water, for example, CNF which has undergone surface modification:

        2.1. Solvent exchange the sample into water via multiple, e.g. 4-7, centrifugation steps.
        2.2. Homogenise the washed sample using the rotor-stator mixer to eliminate the flocculation or aggregation due to the washing process
        2.3. Determine the solid content of the washed suspension by moisture balance and prepare 1% suspension in water. Moisture balance uses the loss on drying (LCD) method. In particular, a liquid or solid sample is placed in a sample pan and an integrated balance weighs the initial sample and then a heating element evaporates the water. The final reading is the percent solids in the initial sample, which indicates how much the sample should be diluted for the required concentration.
        2.4. Place the suspension in a 50ml falcon tube

    3. The falcon tube was placed in the centrifuge. A Sorvall™ RC 6 Plus Centrifuge (Thermo Scientific) was used in this example. The sample was centrifuged at 900g for 30mins.

    4. After the centrifugation, the falcon tube was removed and the top liquid phase was poured off without disrupting the bottom one to ensure there was no free flow liquid at the top of the tube

    5. Three aluminium trays were prepared and their weight recorded as $W_{empty\ pan}$

6. The remaining material in the falcon tube was split into the three trays and the trays weighed and recorded as $W_{empty\ pan+wet\ sample}$

7. The trays were placed in the oven at 105°C for overnight

8. The trays were weighed again and recorded as $W_{empty\ pan+dry\ sample}$

9. The WRV (%) result was then calculated using the equation below:

$$WRV = \frac{W_{empty\ pan+wet\ sample} - W_{empty\ pan+dry\ sample}}{W_{empty\ pan+dry\ sample} - W_{empty\ pan}} \times 100$$

A comparison of the cellulose nanofibrils formed by this method, together with cellulose nanofibrils derived from wood pulp from trees and engineered from bacteria is provided in Table 2 below.

Table 2:

| | Tree/wood (pulp) | Bacteria | Seaweed |
|---|---|---|---|
| Aspect ratio | 10 | >100 | >100 |
| Water Retention Value (%) | 5000 | 1000 | 7000 |
| Energy consumption (kwh/tonne) | >2000 | Very high (> 10,000?) | Very low <200 |
| Time to CNF from source (days) | 7+ | 21+ | +/- 1 |
| Charged dispersible product | Yes | No | Yes or No as required |
| Biocompatibility | No | Yes | Yes |
| Value end product | Low-medium | Very high | Very high |
| Yield from raw material | Low | Low | High (~ 100%) |

As is shown, the resulting cellulose nanofibrils are more akin to CNF derived from high cost bacterial processes, thus providing a high quality product at a much lower production cost and in a much shorter time frame from source to end product. In addition, the seaweed derived CNF offers far greater water retention than is achieved for CNF derived from either wood pulp or bacterial processes.

Further, the yield of CNF from seaweed is significantly greater than that for wood pulp or bacteria, achieving close to 100%.

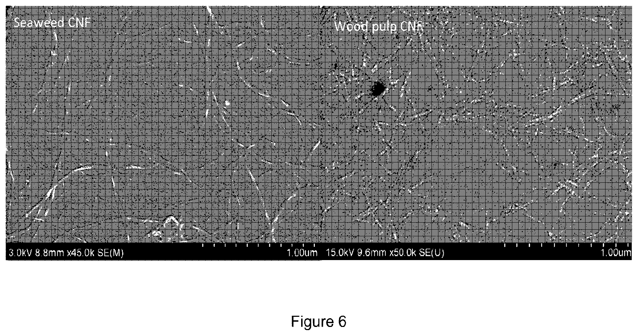

Figure 6 shows SEM images comparing cellulose nanofibrils derived from seaweed (on the left) to cellulose nanofibrils derived from wood pulp (on the right). It is readily apparent from these images that the seaweed derived CNF has a much higher aspect ratio than the wood pulp derived CNF and so provides a much higher quality end product.

In a further embodiment of the present invention, the cellulose nanofibrils resulting from the above method may be further processed to produces cellulose nanocrystals.

The cellulose nanofibrils are subjected to acid hydrolysis using hydrochloric acid or a strong oxidising agent to produce cellulose nanocrystals, which are subsequently extracted from the acid solution by centrifugation and washing.

In particular, unmodified CNC, oxidised CNC and sulphated CNC were produced using hydrochloric acid, ammonium persulphate, and sulphuric acid, in accordance with methods known in the art. The reactions were left for sufficient time to obtain the modified or unmodified CNC but stopped before complete hydrolysis occurred.

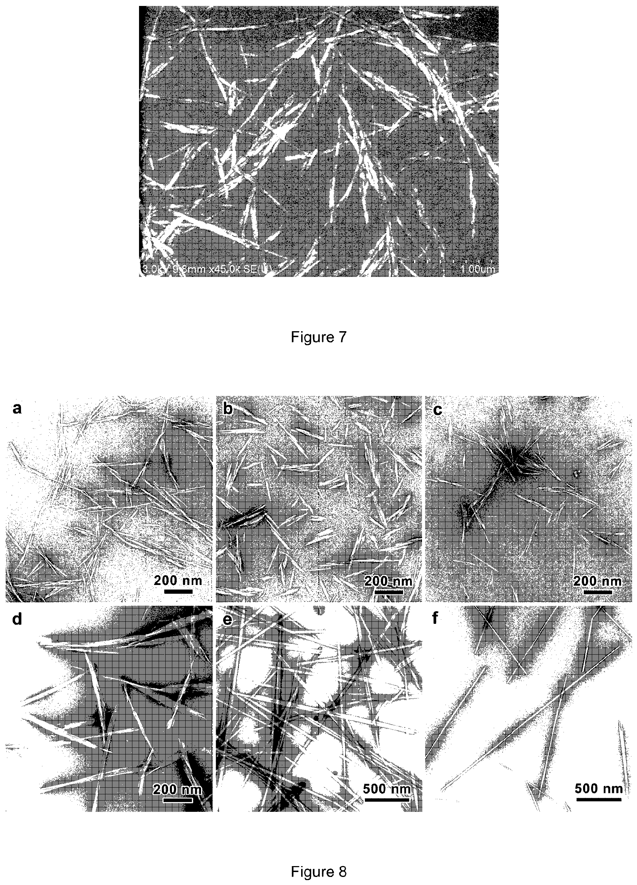

The yield of the resulting material cellulose nanocrystals is approximately 70%, as compared to yields of less than 30% which are achieved for acid hydrolysis of wood pulp derived cellulose nanofibrils. The cellulose nanocrystals are a high quality end product comparable to cellulose nanocrystals derived from bacterial sources. As can be seen from Figures 7 and 8, the nanocrystals formed by the method of the present invention (Fig. 7) are large crystals similar in size and morphology to bacterial synthesised nanocellulose (Fig. 8d). The cellulose nanocrystals derived from wood (Fig. 8a), cotton (Fig. 8b) and bamboo (Fig. 8c) are much smaller crystals, which are of inferior quality, and the cellulose nanocrystals derived from Glaucophyte algae via microalgae synthesis (Fig. 8e) and tunicate (Fig. 8f) are larger crystals. While the high costs of the bacterial derived CNC synthesis and the high costs and difficulties in culturing Glaucophyte algae and tunicates render such processes suitable only for high value commercial end products, CNC derived from the method of the present invention is a more commercial viable product. The resulting CNC can therefore be used in a range of applications without any cost prohibition.

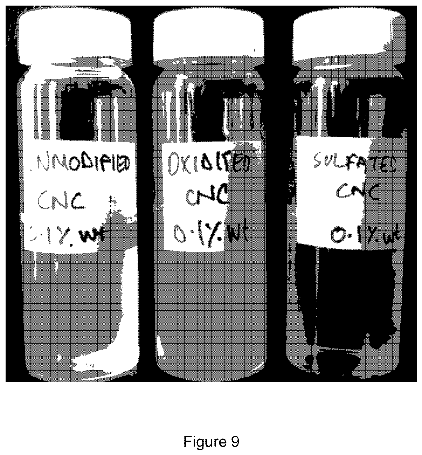

Due to the high yield and high quality of cellulose nanocrystals achieved via acid hydrolysis of cellulose nanofibrils derived from algae via the method of the present invention, further processing of the CNC is efficient and effective, making such processing an economically viable option. For example, as shown in Table 3 below and in Figure 9, CNC derived from acid hydrolysis (using hydrochloric acid) of the cellulose nanofibrils derived from seaweed via the method of the present invention for a reaction time of 4 hours, namely unmodified CNC, has no surface modification and has a low zeta potential. Subsequent oxidisation of the CNC with ammonium persulphate provides an oxidised CNC which has a carboxyl group surface modification and a higher zeta potential. Alternatively, if the cellulose nanofibrils undergo acid hydrolysis using with sulphuric acid for a reaction time of 2 hours, a sulfated CNC is produced which has a sulfate ester group modification with a significantly higher zeta potential.

Table 3:

| Product | Zeta potential mV | Surface modification |
| --- | --- | --- |
| Unmodified CNC | 26.2 | None (native hydroxyl) |
| Oxidised CNC | 35.2 | Carboxyl group |
| Sulfated CNC | 56.6 | Sulfate ester group |

The zeta potentials were derived directly from zeta potential measurements using electrophoretic light scattering and the surface charge moieties were confirmed by FTIR.

Therefore, the cellulose nanofibrils of the present invention are amenable to acid hydrolysis to form cellulose nanocrystals and subsequent surface modification by techniques known in the art. Further, such products are comparable with bacterial derived CNC, thus providing a high quality product at a much lower cost.

Nanocellulose (CNF and CNC) produced by the method of the present invention is naturally uncharged but can be easily modified to requirements, giving flexibility in applications for polar and non-polar environments. For example, a charge may be applied to CNF via a mild oxidation reaction, as is known in the art and discussed in further detail above. As with CNC, CNF derived from the method of the present invention is much more amenable to chemical modification than CNF derived from other sources (e.g. wood pulp) and so such modification reactions require less oxidant and much shorter reaction times to achieve comparable products, thus providing more economically viable methods.

The method of the present invention is faster, cleaner and much cheaper than existing higher plant or bacterial derived cellulose processes. In particular, the time frame from source to CNF may be as little as 3 days and less than a day when the method is scaled up. Further, the method may be a single step high-shear process which allows for ease of scale up via bolt-on modular technology to existing seaweed processing and alginate extraction facility. Further, no harsh chemicals are required and seaweed cellulose is currently a by-product from alginate production. As such the energy requirements for production are at least an order of magnitude lower than with any existing process.

The CNF produced is more homogeneous than with existing methodologies and exhibits certain desirable characteristics, namely, high aspect ratio, highly absorbent fibres. Initial characterisations show this product to be of a higher quality by all standard metrics than any other CNF product currently available.

**Claims**

1. A method for processing isolated fibrous cellulosic material from brown seaweed comprising:

    i) suspending the isolated fibrous cellulosic material in water to form a suspension; and
    ii) passing the suspension through a high shear homogeniser having at least one chamber with a gap of 70 microns to 250 microns, to produce cellulose nanofibrils;
    wherein the isolated fibrous cellulosic material is derived from fibrous cellulosic material extracted from brown seaweed together with alginate and subsequently separated from alginate and the isolated fibrous cellulosic material is not subjected to any pre-treatments to aid extraction of the cellulose nanofibrils, such as treatments with swelling, hydrolytic or oxidation agents prior to suspending in water, as required in known cellulose nanofibril production from wood-pulp; and
    wherein the suspension undergoes up to 7 passes through the at least one chamber.

2. The method of claim 1 wherein the isolated fibrous cellulosic material is suspended in water at a concentration of from 0.5 to 5% by weight, preferably from 1 to 3% by weight.

3. The method of claim 1 or claim 2, wherein the suspension undergoes a single pass through the at least one chamber.

4. The method of claim 1 or claim 2, wherein the suspension undergoes up to 5 passes through the at least one chamber, preferably up to 3 passes through the at least one chamber.

5. The method of any one of the preceding claims wherein the suspension is passed through the at least one chamber at a pressure up to 200MPa.

6. The method of any one of the preceding claims wherein the water is removed from the suspension comprising the cellulose nanofibrils to produce cellulose nanofibrils in a dry form.

7. The method of any one of the preceding claims further comprising the step of subjecting the cellulose nanofibrils to acid hydrolysis to form cellulose nanocrystals.

8. The method of claim 7 wherein the acid hydrolysis uses hydrochloric acid.

9. The method of any one of the preceding claims wherein the cellulose nanofibrils or cellulose nanocrystals undergo surface modification.

10. Cellulose nanofibrils derived from brown seaweed having an aspect ratio of greater than 100 and being a substantially homogeneous product, wherein at least 95% of the cellulose nanofibrils are discrete entities each with two dimensions in the nano range.

11. The cellulose nanofibrils of claim 10 having a water retention of greater than 5000%.

12. A rheological modifier, a medical composite, a single use plastic replacement product, a medical implant or a medical mesh comprising at least one of the cellulose nanofibrils of any one of claims 10 to 11.

13. A method of producing a medical implant or a medical mesh comprising 3-D printing of at least one of the cellulose nanofibrils of any one of claims 10 to 11.

14. Use of the cellulose nanofibrils of any one of claims 10 to 11 in composites, oil and gas, paints, cosmetics, foods, coatings or films.


**Patentansprüche**

1. Ein Verfahren zum Verarbeiten von isoliertem faserigen Zellulosematerial aus Braunalge, das Folgendes beinhaltet:

i) Suspendieren des isolierten faserigen Zellulosematerials in Wasser, um eine Suspension zu bilden; und
ii) Führen der Suspension durch einen Hochscher-Homogenisator mit mindestens einer Kammer mit einer Lücke von 70 Mikrometern bis 250 Mikrometern, um Zellulosenanofasern herzustellen;
wobei das isolierte faserige Zellulosematerial aus faserigem Zellulosematerial erlangt wird, das aus Braunalge gemeinsam mit Alginat extrahiert und anschließend von dem Alginat getrennt wurde und das isolierte faserige Zellulosematerial keinen Vorbehandlungen unterzogen wird, um bei der Extraktion der Zellulosenanofasern zu helfen, wie Behandlungen mit quellenden, hydrolytischen oder Oxidationsmitteln vor dem Suspendieren in Wasser, wie erforderlich bei der bekannten Zellulosenanofaserproduktion aus Holzfaserstoff; und
wobei die Suspension bis zu 7 Führungen durch die mindestens eine Kammer unterzogen wird.

2. Verfahren gemäß Anspruch 1, wobei das isolierte faserige Zellulosematerial in Wasser in einer Konzentration von 0,5 bis 5 Gew.-%, vorzugsweise 1 bis 3 Gew.-% suspendiert wird.

3. Verfahren gemäß Anspruch 1 oder Anspruch 2, wobei die Suspension einer einzigen Führung durch die mindestens eine Kammer unterzogen wird.

4. Verfahren gemäß Anspruch 1 oder Anspruch 2, wobei die Suspension bis zu 5 Führungen durch die mindestens eine Kammer, vorzugsweise bis zu 3 Führungen durch die mindestens eine Kammer, unterzogen wird.

5. Verfahren gemäß einem der vorhergehenden Ansprüche, wobei die Suspension mit einem Druck von bis zu 200 MPa durch die mindestens eine Kammer geführt wird.

6. Verfahren gemäß einem der vorhergehenden Ansprüche, wobei das Wasser von der Suspension, die die Zellulosenanofasern beinhaltet, entfernt wird, um Zellulosenanofaser in Trockenform herzustellen.

7. Verfahren gemäß einem der vorhergehenden Ansprüche, das ferner den Schritt beinhaltet, die Zellulosenanofasern Säurehydrolyse zu unterziehen, um Zellulosenanokristalle zu bilden.

8. Verfahren gemäß Anspruch 7, wobei die Säurehydrolyse Salzsäure verwendet.

9. Verfahren gemäß einem der vorhergehenden Ansprüche, wobei die Zellulosenanofasern oder Zellulosenanokristalle Oberflächenmodifizierung unterzogen werden.

10. Aus Braunalge erlangte Zellulosenanofasern mit einem Seitenverhältnis von mehr als 100, und die ein im Wesentlichen homogenes Produkt sind, wobei mindestens 95 % der Zellulosenanofasern getrennte Einheiten mit jeweils zwei Dinmensionen Nanobereich sind.

11. Zellulosenanofasern gemäß Anspruch 10, die eine Wasserretention von mehr als 5000 % aufweisen.

12. Ein Rheologiemodifikator, ein medizinischer Verbundstoff, ein Einwegkunststoffersatzprodukt, ein medizinisches Implantat oder ein medizinisches Mesh, beinhaltend mindestens eine der Zellulosenanofasern gemäß einem der Ansprüche 10 bis 11.

13. Ein Verfahren zum Herstellen eines medizinischen Implantats oder eines medizinischen Meshs, das 3-D-Drucken von mindestens einer der Zellulosenanofasern gemäß einem der Ansprüche 10 bis 11 beinhaltet.

14. Verwendung der Zellulosenanofasern gemäß einem der Ansprüche 10 bis 11 in Verbundstoffen, Öl und Gas, Farben, Kosmetika, Nahrungsmitteln, Beschichtungen oder Filmen.

**Revendications**

1. Un procédé pour traiter une matière cellulosique fibreuse isolée provenant d'algues brunes comprenant le fait :

   i) de mettre en suspension la matière cellulosique fibreuse isolée dans de l'eau afin de former une suspension ; et
   ii) de faire passer la suspension à travers un homogénéisateur à cisaillement élevé ayant au moins une chambre avec un espace de 70 microns à 250 microns, afin de produire des nanofibrilles de cellulose ;
   dans lequel la matière cellulosique fibreuse isolée est dérivée de matière cellulosique fibreuse extraite d'algues brunes conjointement avec de l'alginate et par la suite séparée de l'alginate et la matière cellulosique fibreuse isolée n'est soumise à aucun prétraitement facilitant l'extraction des nanofibrilles de cellulose, tels que des traitements à l'aide d'agents de gonflement, hydrolytiques ou d'oxydation avant de mettre en suspension dans de l'eau, tel que cela est requis lors de la production connue de nanofibrilles de cellulose à partir de pâte de bois ; et
   dans lequel la suspension subit jusqu'à 7 passages à travers l'au moins une chambre.

2. Le procédé de la revendication 1 dans lequel la matière cellulosique fibreuse isolée est mise en suspension dans de l'eau à une concentration allant de 0,5 à 5 % en poids, de préférence allant de 1 à 3 % en poids.

3. Le procédé de la revendication 1 ou de la revendication 2, dans lequel la suspension subit un seul passage à travers l'au moins une chambre.

4. Le procédé de la revendication 1 ou de la revendication 2, dans lequel la suspension subit jusqu'à 5 passages à travers l'au moins une chambre, de préférence jusqu'à 3 passages à travers l'au moins une chambre.

5. Le procédé de l'une quelconque des revendications précédentes dans lequel la suspension est passée à travers l'au moins une chambre à une pression allant jusqu'à 200 MPa.

6. Le procédé de l'une quelconque des revendications précédentes dans lequel l'eau est retirée de la suspension comprenant les nanofibrilles de cellulose afin de produire des nanofibrilles de cellulose sous forme sèche.

7. Le procédé de l'une quelconque des revendications précédentes comprenant en outre l'étape consistant à soumettre les nanofibrilles de cellulose à une hydrolyse acide afin de former des nanocristaux de cellulose.

8. Le procédé de la revendication 7 dans lequel l'hydrolyse acide utilise l'acide chlorhydrique.

9. Le procédé de l'une quelconque des revendications précédentes dans lequel les nanofibrilles de cellulose ou les nanocristaux de cellulose subissent une modification de surface.

10. Des nanofibrilles de cellulose dérivées d'algues brunes présentant un rapport d'aspect supérieur à 100 et étant un produit substantiellement homogène, au moins 95 % des nanofibrilles de cellulose étant des entités distinctes ayant chacune deux dimensions dans la plage nano.

11. Les nanofibrilles de cellulose de la revendication 10 présentant une rétention d'eau supérieure à 5 000 %.

12. Un modificateur de rhéologie, un composite médical, un produit de remplacement de plastique à usage unique, un implant médical ou une maille médicale comprenant au moins une des nanofibrilles de cellulose de l'une quelconque des revendications 10 à 11.

13. Un procédé consistant à produire un implant médical ou une maille médicale comprenant le fait d'imprimer en 3D au moins une des nanofibrilles de cellulose de l'une quelconque des revendications 10 à 11.

14. Une utilisation des nanofibrilles de cellulose de l'une quelconque des revendications 10 à 11 dans des composites, le pétrole et le gaz, des peintures, des produits cosmétiques, des produits alimentaires, des revêtements ou des films.

A)

B)

C)

D)

E)

F)

Figure 1

**Storage modulus vs No. passes**

Figure 2a

**Storage modulus vs no. passes**

Figure 2b

Figure 3a

Figure 3b

Figure 3c

Figure 4a

| LAB EXTRACTION | MBL SCOTLAND | MBL ICELAND | ASSIGNMENT |
|:---:|:---:|:---:|:---:|
| 3341 | 3339 | 3339 | OH |
| 2895 | 2894 | 2894 | CH |
| 1640 | 1637 | 1638 | OH bending of absorbed $H_2O$ |
| 1427 | 1427 | 1427 | $CH_2$ bending at C-6 |
| 1370 | 1365 | 1364 | CH |
| 1315 | 1314 | 1315 | $CH_2$ wagging at C-6 |
| 1204 | 1203 | 1204 | COH in plane at C-6 |
| 1161 | 1160 | 1160 | COC at βGlycosidic linkage |
| 1108 | 1107 | 1107 | Ring in plane |
| 1054 | 1053 | 1053 | CO at C3 |
| 1031 | 1028 | 1029 | CO at C-6 |
| 898 | 897 | 898 | COC at βGlycosidic linkage |
| 663 | 662 | 663 | COH out of plane |

Figure 4b

Figure 5a

Figure 5b

Figure 6

Figure 7

Figure 8

Figure 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **JIAQI GUO et al.** Contribution of Residual Proteins to the Thermomechanical Performance of Cellulosic Nanofibrils Isolated from Green Macroalgae. *ACS Sustainable Chemistry & Engineering,* 07 August 2021, vol. 5, 6978-6985

- **MOON ROBERT J. et al.** Cellulose Nanomaterials Review: Structure, Properties and Nanocomposites. *Chemical Society Reviews,* 01 January 2011, vol. 40 (7), 3941
- **CARRILLO et al.** Microemulsion Systems for Fiber Deconstruction into Cellulose Nanofibrils. *ACS Applied Materials and Interfaces,* 2014, vol. 6, 22622-22627